# EUROPÄISCHE PATENTSCHRIFT

(11) **EP 3 038 546 B1**
(45) Veröffentlichungstag und Bekanntmachung des Hinweises auf die Patenterteilung: **15.01.2020**
(21) Anmeldenummer: 14757919.7
(22) Anmeldetag: 28.08.2014
(51) Int. Cl.: A61B 17/221

(54) **SCHLINGENVORRICHTUNG ZUM EINFANGEN EINES OBJEKTS**
SNARE DEVICE FOR CAPTURING AN OBJECT
DISPOSITIF D'ANSE POUR SAISIR UN OBJET

(30) Priorität: 28.08.2013 DE 202013103881 U
(43) Veröffentlichungstag der Anmeldung: 06.07.2016
(73) Patentinhaber: PFM Medical AG, 50996 Köln (DE)
(72) Erfinder: KRAMANN, Bernhard, 66424 Homburg/Saar (DE)
(74) Vertreter: Hohendorf, Carsten
(86) Internationale Anmeldenummer: PCT/EP2014/068271
(87) Internationale Veröffentlichungsnummer: WO 2015/028555

(56) Entgegenhaltungen:
- EP-A1- 1 113 755
- EP-A1- 2 052 688
- DE-A1-102006 053 448
- US-A1- 2013 184 741

## Beschreibung

Die Erfindung betrifft eine Schlingenvorrichtung zum Einfangen eines Objektes, insbesondere zum Einfangen von Objekten oder Fremdkörpern in einem menschlichen oder tierischen Körper, umfassend ein hohles Element, insbesondere einen Katheter, und eine Schlinge aus einem flexiblen elastischen Formgedächtnismaterial, wobei die Schlinge relativ zu dem hohlen Element beweglich und in das hohle Element hineinziehbar und aus diesem herausschiebbar ist, und wobei die Schlinge eine eingeprägte Ursprungsform aufweist, in welche die Schlinge beim Herausschieben aus dem hohlen Element durch die beim Hereinziehen herbeigeführten Materialspannungen zurückkehrt, wobei die Ursprungsform bei der Schlinge einen Verwindungsbereich aufweist, wodurch eine erste Schlaufe und eine zweite Schlaufe ausgebildet sind, wobei die erste Schlaufe im Wesentlichen parallel zu der Längsachse des hohlen Elementes angeordnet ist und die zweite Schlaufe gegenüber der Längsachse des hohlen Elementes abgewinkelt ist.

Schlingenvorrichtungen zum Einfangen von Objekten aus Formgedächtnismaterial, welche in einem hohlen Element gelagert und beweglich gegenüber diesem ausgebildet sind, sind aus dem Stand der Technik bekannt.

In der DE 195 14 534 C2 und der EP 1 113 755 B1 sind Fangschlingen beschrieben, welche mittels eines minimalinvasiven Eingriffs das Extrahieren von Objekten aus dem menschlichen oder tierischen Körper ermöglichen. Diese weisen jeweils eine Schlinge aus Formgedächtnismaterial auf, welche um ein zu extrahierendes Objekt gelegt werden kann. Ist das einzufangende Objekt innerhalb der Schlinge angeordnet, wird die Schlinge zugezogen und das Objekt kann extrahiert werden. Mit derartigen Schlingenvorrichtungen können Objekte, insbesondere Fragmente von Kathetern oder von Führungsdrähten aus vielen unterschiedlichen Richtungen eingefangen werden. Voraussetzung hierfür ist, dass sich das Objekt mit der Schlinge umfassen lässt. Hierzu muss das Objekt ein zugängliches Ende aufweisen, über welches die Schlinge gestülpt werden kann, so dass dieses sondierbar ist. Zum Einfangen von Objekten mit nicht-sondierbaren Enden können sie nicht verwendet werden kann.

Sondierbar im Sinne der Erfindung bedeutet, dass um ein zugängliches Ende eines einzufangenden Objektes die Schlinge der Schlingenvorrichtung gelegt wird. Anschließend wird die Schlinge zugezogen, so dass das Objekt von der Schlinge umfasst ist. Durch ein Zurückziehen der Schlingenvorrichtung wird abschließend das Objekt extrahiert. Der Begriff "sondierbar" wird im Folgenden in dieser beschriebenen Weise verwendet.

Des Weiteren beschreiben die EP 2 052 688 B1 und die DE 10 2006 053 448 A1 Schlingenvorrichtungen, welche einen Riegeldraht aufweisen, der durch die Schlinge der Schlingenvorrichtung geschoben werden kann, um ein Objekt zwischen einem Teil der Schlinge und dem sich durch die Schlinge erstreckenden Riegeldraht einzufangen. Mit diesen Schlingenvorrichtungen können Objekte eingefangen werden, deren Enden nicht sondierbar sind, indem die Schlinge neben das einzufangende Objekt gelegt wird und der Riegeldraht auf der gegenüberliegenden Seite das Objekt umschließt, wobei der Riegeldraht in die Schlinge eingreift und das Objekt zwischen Schlinge und Riegendraht eingefangen wird. Das Objekt ist folglich eingefangen und kann extrahiert werden. Nachteilig an diesen Schlingenvorrichtungen ist, dass die mit den Schlingenvorrichtungen verwendeten hohlen Elemente einen großen Durchmesser aufweisen, da diese zusätzlich zu der Schlinge noch einen Riegeldraht aufweisen.

Im klinischen Alltag hat sich gezeigt, dass Situationen, in denen die Enden der zu extrahierenden Objekte nicht-sondierbar sind, relativ häufig anzutreffen sind. Bei etwa jedem zehnten Eingriff liegt dieser Fall vor. Mittels eines gesondert einzuführenden Pigtail-Katheters kann versucht werden, sich mit dem Ende des Pigtail-Katheters an dem Objekt, beispielsweise einem Fragment eines Katheters, einzuhaken und anschließend eine Zugbewegung auszuführen, so dass ein Ende des Objektes durch die Verschiebung in einen freiliegenden Bereich, beispielsweise eine benachbarte Vene oder Körperöffnung, sondierbar wird.

Um Objekte, deren Enden nicht sondierbar sind, einzufangen, sind aus dem Stand der Technik spezielle auf diesen Anwendungsfall ausgerichtete Zangen zur Extraktion der Objekte bzw. spezielle Fangschlingen bekannt. Diese speziellen Instrumente sind ausschließlich für den Anwendungsfall von einzufangenden Objekten mit nicht-sondierbaren Enden vorgesehen. Sie weisen spezielle Haken bzw. hakenartige Schlingen auf, mit denen das Objekt angehakt werden kann und eine Fixierung durch einen Riegeldraht erfolgen kann. Der Riegeldraht verschränkt sich zwischen dem Objekt und der hakenartigen Schlinge, so dass das Objekt verriegelt ist und geborgen werden kann.

Nachteilig bei dem aus dem Stand der Technik bekannten Lösungen ist, dass diese speziellen Instrumente zum Einfangen von Objekten mit nicht-sondierbaren Enden erst dann eingesetzt werden, wenn mit einer konventionellen Schlingenvorrichtung das Einfangen fehlgeschlagen ist. Die konventionelle Schlingenvorrichtung muss dann zunächst entfernt werden und das spezielle Instrument gesondert eingeführt werden. Hieraus ergibt sich ein weiterer Nachteil, der darin begründet ist, dass das Kaliber der Spezialinstrumente größer ist als das Kaliber der konventionellen Schlingenvorrichtung. Der Durchmesser des hohlen Elementes ist deutlich größer, was zu Komplikationen an der Punktionsstelle führen kann. Die Folgen können die Entwicklung von Thrombosen und/oder von schwer zu stillenden Nachblutungen sein.

Ausgehend von diesem Stand der Technik besteht die Aufgabe der Erfindung darin, die Leistungsfähigkeit der aus dem Stand der Technik bekannten Schlingenvorrichtung zu verbessern, insbesondere um über das Einfangen von Objekten mit sondierbaren Enden hinausgehend das Einfangen von Objekten mit nicht-sondierbaren Enden zu ermöglichen.

Eine weitere Aufgabe der Erfindung besteht darin, eine Schlingenvorrichtung zu schaffen, deren Kaliber auch bei dem Anwendungsfall des Einfangens von Objekten mit nicht-sondierbaren Enden gering ist, insbesondere um Komplikationen für den Patienten zu vermeiden.

Zur technischen Lösung dieser Aufgabe wird mit der, durch Anspruch 1 definierten Erfindung eine Schlingenvorrichtung der eingangs genannten Art vorgeschlagen, welche dadurch gekennzeichnet ist, dass die Schlinge derart verformt in das hohle Element eingebracht und innerhalb des hohlen Elementes angeordnet ist, dass die zweite Schlaufe relativ zu der ersten Schlaufe und durch die erste Schlaufe hindurch verschwenkt ist, so dass die zweite Schlaufe beim Austreten aus dem hohlen Element durch die dabei entstehende Entlastung des Formgedächtnismaterials durch die erste Schlaufe zurückschwenkt, wodurch sich das Objekt umfassen lässt.

Der Erfindung liegt die Erkenntnis zugrunde, dass Schlingen aus Formgedächtnismaterial bei eingangs genannten Schlingenvorrichtungen die Ursprungsform einnehmen, wenn sie aus dem hohlen Element austreten. Dies geschieht auf Grund der beim Eintritt in das hohle Element hervorgerufenen Materialspannungen in der Schlinge. Wird die Schlinge nun derart verformt in das hohle Element eingebracht, dass die zweite Schlaufe der Schlinge relativ zu der ersten Schlaufe und durch die erste Schlaufe hindurch verschwenkt ist, dann muss die zweite Schlaufe nach dem Austritt aus dem hohlen Element, um die Ursprungsform der Schlinge einzunehmen, wieder zurück durch die erste Schlaufe schwenken. Durch diese Schwenkbewegung lässt sich ein einzufangendes Objekt mittels der Schlinge umfassen. Objekte, deren Enden nicht sondierbar sind, lassen sich folglich mit der erfindungsgemäßen Lösung einfangen und durch das hohle Element bergen und aus dem menschlichen bzw. tierischen Körper entfernen.

Vorteilhafterweise wird die Schlinge derart verformt in das hohle Element eingebracht, dass die erste Schlaufe gegenüber der zweiten Schlaufe um etwa 270° und durch die erste Schlaufe hindurch verschwenkt wird und daher gestreckt und von der distalen Austrittsöffnung des hohlen Elementes wegweisend in das hohle Element eingebracht wird. Dies stellt eine hinreichend lange Schwenkbewegung der zweiten Schlaufe sicher, um ein einzufangendes Objekt mittels der Schlinge vollständig zu umfassen bzw. zu umwickeln.

Die Schlinge aus Formgedächtnismaterial der erfindungsgemäßen Schlingenvorrichtung ist in einem hohlen Element, insbesondere in einem Katheter, gelagert und relativ zu dem Katheter beweglich ausgebildet. Das Ausfahren der Schlinge aus dem Katheter kann über eine Betätigungseinrichtung durch einen Untersucher ausgeführt werden. Beim Austreten aus dem distalen Ende des hohlen Elements entfaltet sich die Schlinge auf Grund der in dem Formgedächtnismaterial vorhandenen Materialspannung. Die Schlinge kann vollständig in das hohle Element zurückgezogen werden, wenn kein Objekt von der Schlinge gefasst bzw. umfasst wurde.

Die erfindungsgemäße Schlingenvorrichtung kann bei allen vaskulären Prozeduren, wie beispielsweise Stentimplantationen, endoskopischen Operationen oder zur Behandlung von kardiovaskulären Krankheiten eingesetzt werden. Grundlegend kann die erfindungsgemäße Schlingenvorrichtung bei jeder Operation eingesetzt werden, die das Einfangen und Bergen von Objekten oder Fremdkörpern in einem menschlichen oder tierischen Körper erforderlich macht. Dies wird erfindungsgemäß mittels eines minimalinvasiven Eingriffes durchgeführt.

Vorteilhafterweise ist in der Ursprungsform der Schlinge die zweite Schlaufe gegenüber der ersten Schlaufe um etwa 90° abgewinkelt. Durch eine derartige Konfiguration der Schlingenvorrichtung kann ein einzufangendes Objekt sicher und einfach mit der Schlinge gefasst werden. Insbesondere ist sicher zu stellen, dass das eingefangene Objekt sicher gehalten wird, so dass es durch das hohle Element geborgen werden kann.

In einer weiteren Ausgestaltung ist in der Ursprungsform der Schlinge die erste Schlaufe größer als die zweite Schlaufe. Dies erleichtert wesentlich das Zurückschwenken der zweiten Schlaufe beim Austreten aus dem hohlen Element.

Vorteilhafterweise ist in der Ursprungsform der Schlinge die erste Schlaufe im Wesentlichen dreiecksförmig ausgebildet. Dies unterstützt ebenfalls das Zurückschwenken der zweiten Schlaufe durch die erste Schlaufe hindurch.

In einer bevorzugten Ausgestaltung weist in der Ursprungsform der Schlinge die erste Schlaufe eine Ausbuchtung auf, welche das Hindurchschwenken der ersten Schlaufe durch die zweite Schlaufe erleichtert. In einer besonders bevorzugten Ausgestaltung bilden in der Ursprungsform der Schlinge die erste Schlaufe und die zweite Schlaufe einen Überlappungsbereich aus, in welchem das einzufangende Objekt beim Umfassen durch die Schlinge gelagert ist. Damit das einzufangende Objekt nicht die Schwenkbewegung der zweiten Schlaufe der Schlinge beeinträchtigt, wird das einzufangende Objekt zwischen den beiden Schlaufen in dem Überlappungsbereich gelagert. Die unmittelbare Folge hiervon ist, dass die zweite Schlaufe frei beweglich ist. Die Ausbuchtung der ersten Schlaufe wirkt beim Umfassen eines einzufangenden Objektes durch die Schlaufen der Schlinge wie ein Scharnier, wobei das einzufangende Objekt beim Zurückschwenken der zweiten Schlaufe durch die erste Schlaufe zwischen den beiden Schlaufen im Überlappungsbereich eingeklemmt wird. Derart kann sichergestellt werden, dass das Objekt vollständig und sicher umfasst wird, so dass eine Bergung durch das hohle Element möglich ist.

In einer weiteren Ausgestaltung besteht das Formgedächtnismaterial der Schlinge aus Metall, insbesondere aus Nitinol, oder aus Kunststoff.

Vorteilhafterweise weist das hohle Element einen Durchmesser von etwa 2 bis etwa 8 French auf, bevorzugt von etwa 4 bis etwa 6 French. Der Durchmesser des mit der Schlingenvorrichtung verwendeten hohlen Elements ist folglich signifikant geringer, als der Durchmesser der aus dem Stand der Technik bekannten Spezialinstrumente zum Einfangen von Objekten bzw. Fremdkörpern mit nicht-sondierbaren Enden. Ein wesentlicher Vorteil hierbei ist, dass Komplikationen durch die Verwendung von hohlen Elementen mit größeren Durchmessern an der Punktionsstelle vermieden werden können. Insbesondere die Gefahr des Auftretens von Thrombosen und/oder schwer zu stillenden Nachblutungen an der Punktionsstelle wird vermindert.

Die Schlingenvorrichtung weist vorteilhafterweise an der Schlinge eine Markierung auf, um die Schlinge unter Darstellungsvorrichtungen sichtbar zu machen. Während der Durchführung eines Eingriffes, um ein Objekt oder einen Fremdkörper zu bergen, ist es vorteilhaft, wenn insbesondere die zweite Schlaufe eine Markierung aufweist, die unter einer Darstellungsvorrichtung die Schlinge sichtbar macht. Möglich ist dies beispielsweise durch die Verwendung eines Fluoroskops, eines MRT bzw. weiteren elektronischen Darstellungsvorrichtungen, die Markierungen mittels eines Röntgengerätes oder einer Sonografie an der Schlinge sichtbar machen. Die Markierungen können beispielsweise Magnetresonanzmarkierungen sein.

Weitere Einzelheiten, Merkmale und Vorteile der Erfindungen werden nachfolgend anhand des in den Figuren dargestellten Ausführungsbeispiels näher erläutert. Dabei zeigt:
- Fig. 1: eine erfindungsgemäße Schlingenvorrichtung in der Ursprungsform der Schlinge und ausgetreten aus einem Katheter;
- Fig. 2: die erfindungsgemäße Schlingenvorrichtung, welche ein einzufangendes Objekt mit nicht-sondierbaren Enden umfasst hat und
- Fig. 3: intrakardiale Lagen von einzufangenden Objekten mit nichtsondierbaren Enden, die typische Anwendungsfälle für die erfindungsgemäße Schlingenvorrichtung darstellen.

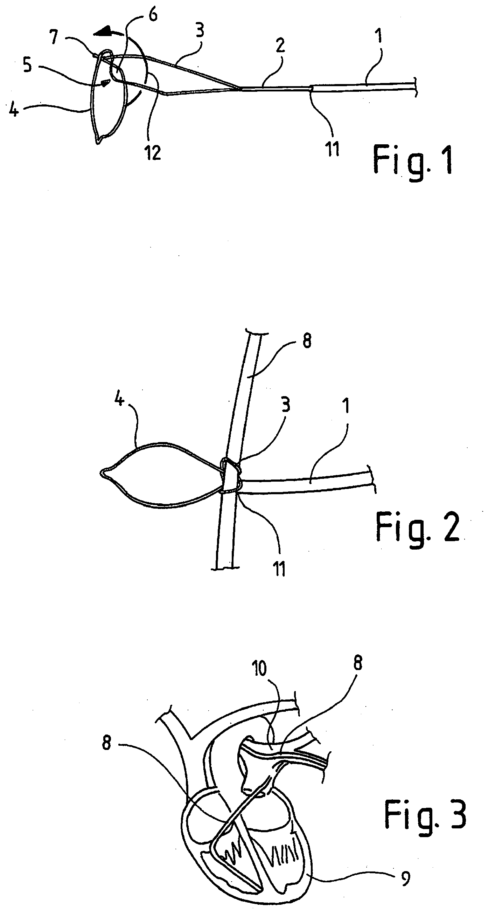

Fig. 1 zeigt eine erfindungsgemäße Schlingenvorrichtung zum Einfangen von Objekten, insbesondere zum Einfangen von Objekten oder Fremdkörpern in einem menschlichen oder tierischen Körper. Die Schlingenvorrichtung weist einen Katheter 1 und eine Schlinge auf. Die Schlinge besteht aus einem flexiblen, elastischen Formgedächtnismaterial, wobei die Schlinge relativ zu dem Katheter 1 beweglich und in den Katheter hineinziehbar und aus diesem herausschiebbar ist. Ausgetreten aus dem Katheter 1 nimmt die Schlinge eine eingeprägte Ursprungsform ein. Dies geschieht beim Herausschieben der Schlinge aus dem Katheter 1 durch die beim Hereinziehen in den Katheter herbeigeführten Materialspannungen in dem Formgedächtnismaterial der Schlinge. Die Schlinge weist eine erste Schlaufe 3 und eine zweite Schlaufe 4 auf, die über einen Verwindungsbereich 7 miteinander verbunden sind. Am distalen Ende 11 des Katheters 1 ist die Schlinge einer Ummantelung 2 versehen. Die erste Schlaufe 3 ist im Wesentlichen dreiecksförmig ausgebildet und bildet mit der zweiten Schlaufe 4 einen Überlappungsbereich 6. Ferner weist die erste Schlaufe 3 eine Ausbuchtung 5 auf.

Die Schlinge ist durch Ziehen bzw. Drücken am proximalen Ende des Katheters relativ zu diesem bewegbar. Dazu kann ein Untersucher an der Ummantelung 2 der Schlinge bzw. an zumindest einem Draht der Schlinge ziehen bzw. drücken. Durch diese Bewegung der Schlinge ist ein zu extrahierendes Objekt einfangbar. Um ein eingefangenes Objekt aus dem menschlichen oder tierischen Körper zu extrahieren, wird am proximalen Ende des Katheters 1 an der Ummantelung 2 bzw. an zumindest einem Draht der Schlinge gezogen. Durch das Ziehen wird die Schlinge wenigstens teilweise in den Katheter 1 gezogen und dahingehend verändert, dass sich die Schlinge um das einzufangende Objekt festzieht. Falls das einzufangende Objekt kleiner ist als die Öffnung des verwendeten Katheters 1, kann das Objekt durch den Katheter 1 geborgen bzw. aus dem menschlichen oder tierischen Körper entfernt werden.

Um erfindungsgemäß ein Objekt, beispielsweise ein Fragment eines Katheters, mit nicht-sondierbaren Enden einfangen zu können, muss ausgehend von der Darstellung in Fig. 1 die Ursprungsform verändert werden und die Schlinge in der veränderten Form in den Katheter 1 eingebracht werden. Hierzu wird die zweite Schlaufe 4 um etwa 270° relativ zu der ersten Schlaufe 3 und durch die erste Schlaufe 3 durch verschwenkt. Diese Schwenkung ist in der Zeichnung durch den Pfeil mit dem Bezugszeichen 12 dargestellt. Auf diese Weise werden die erste Schlaufe 3 und die zweite Schlaufe 4 gestreckt in den Katheter 1 eingeführt. Beim anschließenden Austreten der Schlinge ist durch die dabei entstehende Entlastung des Formgedächtnismaterials der Schlinge die zweite Schlaufe 4 danach bestrebt, in die in Figur 1 dargestellte Ursprungsform der Schlinge zurückzukehren. Dies gelingt nur, wenn sie durch die dreiecksförmig ausgebildete erste Schlaufe 3 zurückschwenkt. Es hat sich gezeigt, dass in manchen Fällen das einzufangende Objekt die Drehbewegung die zweite Schlaufe 4 behindern kann. Um dies zu vermeiden, kann die erste Schlaufe 3 eine Ausbuchtung 5 aufweisen und mit der zweiten Schlaufe 4 den Überlappungsbereich 6 ausbilden. In der Ausbuchtung 5 entsteht eine Nische, in der das einzufangende Objekt gelagert wird. Wenn nun die zweite Schlaufe 4 durch die erste Schlaufe 3 zurückschwenkt, wird das einzufangende Objekt in dem Überlappungsbereich 6 eingeklemmt. Die Ausbuchtung 5 wirkt dabei wie ein Scharnier. Die Drehbewegung der zweiten Schlaufe 4 wird durch das einzufangende Objekt nicht behindert und das Objekt lässt sich umfassen.

In Fig. 2 ist ein Objekt 8 mit nicht-sondierbaren Enden von der erfindungsgemäßen Schlingenvorrichtung eingefangen. Die zweite Schlaufe 4 weist nach dem Einfangen eine im Wesentlichen ovale Form auf. Die erste Schlaufe 3 ist um das Objekt 8 herumgeschlungen. Wenn nun das Objekt 8 von der Schlinge umfasst ist, wird das distale Ende 11 des Katheters 1 vorgeschoben, wobei die Position des Objektes 8 unverändert bleibt. Durch das Vorschieben des Katheters 1 zieht sich die Schlinge um das Objekt 8 fest und das Objekt 8 kann extrahiert werden.

In Fig. 3 sind Lagen von Objekten mit nicht-sondierbaren Enden dargestellt. Diese Lagen bilden typische Anwendungsfälle für die erfindungsgemäße Schlingenvorrichtung. Vorliegend befindet sich ein Objekt 8 in der Pulmonalarterie 10 und liegt derart ungünstig, dass die Enden des Objektes 8 nicht sondierbar sind. Eine vergleichbar ungünstige Lage eines einzufangenden Objektes 8 liegt vor, wenn sich dieses in einer intrakardialen Lage im Herzen 9 befindet. Auch dann sind die Enden des einzufangenden Objektes 8 nicht sondierbar. Für derartige Situationen wurden bisher Spezialinstrumente zur Extraktion verwendet. Erfindungsgemäß kann, wie oben beschrieben wurde, eine erfindungsgemäße Schlingenvorrichtung eingesetzt werden.

Vorteilhafterweise weist der mit einer erfindungsgemäßen Schlingenvorrichtung verwendete Katheter einen sehr viel geringeren Durchmesser auf, als die Katheter der aus dem Stand der Technik bekannten Spezialinstrumente. Ein weiterer Vorteil der vorliegenden Lösung ist, dass mittels der erfindungsgemäßen Schlingenvorrichtung ebenfalls Objekte mit sondierbaren Enden einfangbar sind. Der Einsatz der Schlingenvorrichtung gestaltet sich dann identisch zu der Vorrichtung und dem Verfahren, welches in der EP 2 052 688 B1 beschrieben ist.

Das in den Figuren dargestellte und beschriebene Ausführungsbeispiel dient lediglich der Erläuterung der Erfindung und ist für diese nicht beschränkend.

### Bezugszeichenliste

- 1: Katheter
- 2: Ummantelung
- 3: erste Schlaufe
- 4: zweite Schlaufe
- 5: Ausbuchtung
- 6: Überlappungsbereich
- 7: Verwindungsbereich
- 8: Objekt
- 9: Herz
- 10: Pulmonalarterie
- 11: distales Ende
- 12: Schwenkung

## Patentansprüche

1. Schlingenvorrichtung zum Einfangen eines Objektes (8), insbesondere zum Einfangen von Objekten (8) oder Fremdkörpern in einem menschlichen oder tierischen Körper, umfassend ein hohles Element (1), insbesondere einen Katheter, und eine Schlinge (3, 4) aus einem flexiblen elastischen Formgedächtnismaterial, wobei die Schlinge (3, 4) relativ zu dem hohlen Element (1) beweglich und in das hohle Element (1) hineinziehbar und aus diesem herausschiebbar ist, und wobei die Schlinge (3, 4) eine eingeprägte Ursprungsform aufweist, in welche die Schlinge (3, 4) beim Herausschieben aus dem hohlen Element (1) durch die beim Hereinziehen herbeigeführten Materialspannungen zurückkehrt, wobei die Ursprungsform bei der Schlinge (3, 4) einen Verwindungsbereich (7) aufweist, wodurch eine erste Schlaufe (3) und eine zweite Schlaufe (4) ausgebildet sind, wobei die erste Schlaufe (3) im Wesentlichen parallel zu der Längsachse des hohlen Elementes (1) angeordnet ist und die zweite Schlaufe (4) gegenüber der Längsachse des hohlen Elementes (1) abgewinkelt ist,
**dadurch gekennzeichnet, dass**
die Schlinge (3, 4) derart verformt in das hohle Element (1) eingebracht und innerhalb des hohlen Elements (1) angeordnet ist, dass die zweite Schlaufe (4) relativ zu der ersten Schlaufe (3) und durch die erste Schlaufe (3) hindurch verschwenkt (12) ist, so dass die zweite Schlaufe (4) beim Austreten aus dem hohlen Element (1) durch die dabei entstehende Entlastung des Formgedächtnismaterials durch die erste Schlaufe (3) zurückschwenkt, wodurch sich das Objekt (8) umfassen lässt.

2. Schlingenvorrichtung nach Anspruch 1, **dadurch gekennzeichnet, dass** in der Ursprungsform der Schlinge (3, 4) die zweite Schlaufe (4) gegenüber der ersten Schlaufe (3) um etwa 90° abgewinkelt ist.

3. Schlingenvorrichtung nach Anspruch 1 oder Anspruch 2, **dadurch gekennzeichnet, dass** in der Ursprungsform der Schlinge (3, 4) die erste Schlaufe (3) größer ist als die zweite Schlaufe (4).

4. Schlingenvorrichtung nach einem der Ansprüche 1 bis 3, **dadurch gekennzeichnet, dass** in der Ursprungsform der Schlinge (3, 4) die erste Schlaufe (3) im Wesentlichen dreiecksförmig ausgebildet ist.

5. Schlingenvorrichtung nach einem der Ansprüche 1 bis 4, **dadurch gekennzeichnet, dass** in der Ursprungsform der Schlinge (3, 4) die erste Schlaufe (3) eine Ausbuchtung (5) aufweist, welche das Hindurchschwenken der ersten Schlaufe (3) durch die zweite Schlaufe (4) erleichtert.

6. Schlingenvorrichtung nach einem der Ansprüche 1 bis 5, **dadurch gekennzeichnet, dass** in der Ursprungsform der Schlinge (3, 4) die erste Schlaufe (3) und die zweite Schlaufe (4) einen Überlappungsbereich (6) ausbilden, in welchem das einzufangende Objekt (8) beim Umfassen durch die Schlinge (3, 4) gelagert ist.

7. Schlingenvorrichtung nach einem der Ansprüche 1 bis 5, **dadurch gekennzeichnet, dass** das Formgedächtnismaterial der Schlinge (3, 4) aus Metall, insbesondere aus Nitinol, oder aus Kunststoff besteht.

8. Schlingenvorrichtung nach einem der Ansprüche 1 bis 7, **dadurch gekennzeichnet, dass** das hohle Element (1) einen Durchmesser von etwa 2 bis etwa 8 French aufweist, bevorzugt von etwa 4 bis etwa 6 French.

9. Schlingenvorrichtung nach einem der Ansprüche 1 bis 8, **dadurch gekennzeichnet, dass** an der Schlinge (3, 4) eine Markierung vorgesehen ist, um die Schlinge (3, 4) unter Darstellungsvorrichtungen sichtbar zu machen.

## Claims

1. Snare device for catching an object (8), in particular for catching objects (8) or foreign bodies in a human or animal body, comprising a hollow element (1), in particular a catheter, and a snare (3, 4) of a flexible material, wherein the snare (3, 4) is movable relative to the hollow element (1) and can be pulled into the hollow element (1) and pushed out of it, and wherein the snare (3, 4) has an impressed original shape to which said snare (3, 4) returns, when pushed out from the hollow element (1), as a result of the material stresses caused when it was pulled in, wherein the original shape of the snare (3, 4) has a twisted area (7), as a result of which a first loop (3) and a second loop (4) are formed, wherein the first loop (3) is arranged substantially parallel to the longitudinal axis of the hollow element (1), and the second loop (4) is at an angle with respect to the longitudinal axis of the hollow element (1),
**characterized in that**
the snare (3, 4) is introduced such deformed into the hollow element (1) and is arranged inside the hollow element (1), that the second loop (4) is pivoted (12) relative to the first loop (3) and through the first loop (3), such that the second loop (4), on emerging from the hollow element (1), pivots back through the first loop (3) as a result of the load being removed from the shape-memory material, thereby allowing the object (8) to be enclosed.

2. Snare device according to claim 1, **characterized in that** in the original shape of the snare (3, 4) the second loop (4) is at an angle of about 90° with respect to the first loop (3).

3. Snare device according to claim 1 or claim 2, **characterized in that** in the original shape of the snare (3, 4) the first loop (3) is larger than the second loop (4).

4. Snare device according to one of claims 1 to 3, **characterized in that** in the original shape of the snare (3, 4) the first loop (3) is substantially triangular formed.

5. Snare device according to one of claims 1 to 4, **characterized in that** in the original shape of the snare (3, 4) the first loop (3) has a bulge (5), which facilitates the through-pivoting of the first loop (3) through the second loop (4).

6. Snare device according to one of claims 1 to 5, **characterized in that** in the original shape of the snare (3, 4) the first loop (3) and the second loop (4) form an overlapping area (6), in which the object (8) to be caught is supported during the enclosure by the snare (3, 4).

7. Snare device according to one of claims 1 to 5, **characterized in that** the shape-memory material of the snare (3, 4) is formed of shape-memory metal or plastic.

8. Snare device according to one of claims 1 to 7, **characterized in that** the hollow element (1) has a diameter of about 2 to about 8 French, in particular of about 4 to about 6 French.

9. Snare device according to one of claims 1 to 8, **characterized in that** the snare (3, 4) includes a marker to make the snare (3, 4) visible under a displaying apparatus.

## Revendications

1. Dispositif d'anse pour saisir un objet (8), en particulier pour saisir des objets (8) ou des corps étrangers dans un corps humain ou animal, comprenant un élément creux (1), en particulier un cathéter, et une anse (3, 4) d'un matériau flexible, dans lequel l'anse (3, 4) est mobile par rapport à l'élément creux (1) et peut être tirée dans l'élément creux (1) et poussée à l'extérieur de lui, et dans lequel l'anse (3, 4) présente une forme d'origine imprimée à laquelle revient ladite anse (3, 4), quand elle est poussée à l'extérieur de l'élément creux (1), à la suite des contraintes matérielles provoquées lorsqu'elle a été tirée, dans lequel la forme d'origine de l'anse (3, 4) comporte une zone tordue (7), en conséquence de laquelle une première boucle (3) et une seconde boucle (4) sont formées, dans lequel la première boucle (3) est agencée de manière sensiblement parallèle à l'axe longitudinal de l'élément creux (1), et la seconde boucle (4) est à un angle par rapport à l'axe longitudinal de l'élément creux (1),
**caractérisé en ce que**
l'anse (3, 4) est introduite ainsi déformée dans l'élément creux (1) et est agencée à l'intérieur de l'élément creux (1), que la seconde boucle (4) est pivotée (12) par rapport à la première boucle (3) et à travers la première boucle (3), de telle sorte que la seconde boucle (4), lorsqu'elle sort de l'élément creux (1), pivote en arrière à travers la première boucle (3) à la suite de la charge retirée du matériau à mémoire de forme, permettant ainsi à l'objet (8) d'être enfermé.

2. Dispositif d'anse selon la revendication 1, **caractérisé en ce que**, dans la forme d'origine de l'anse (3, 4), la seconde boucle (4) est à un angle d'environ 90° par rapport à la première boucle (3).

3. Dispositif d'anse selon la revendication 1 ou la revendication 2, **caractérisé en ce que**, dans la forme d'origine de l'anse (3, 4), la premier boucle (3) est plus grande que la seconde boucle (4).

4. Dispositif d'anse selon l'une des revendications 1 à 3, **caractérisé en ce que**, dans la forme d'origine de l'anse (3, 4), la première boucle (3) est sensiblement de forme triangulaire.

5. Dispositif d'anse selon l'une des revendications 1 à 4, **caractérisé en ce que**, dans la forme d'origine de l'anse (3, 4), la premier boucle (3) présente un renflement (5), qui facilite le pivotement traversant de la première boucle (3) à travers la seconde boucle (4).

6. Dispositif d'anse selon l'une des revendications 1 à 5, **caractérisé en ce que**, dans la forme d'origine de l'anse (3, 4), la premier boucle (3) et la seconde boucle (4) forment une zone de chevauchement (6), dans laquelle l'objet (8) à saisir est supporté durant l'enfermement par l'anse (3, 4).

7. Dispositif d'anse selon l'une des revendications 1 à 5, **caractérisé en ce que** le matériau à mémoire de forme de l'anse (3, 4) est formé de métal ou plastique à mémoire de forme.

8. Dispositif d'anse selon l'une des revendications 1 à 7, **caractérisé en ce que** l'élément creux (1) présente un diamètre d'environ 2 à environ 8 Ch, en particulier d'environ 4 à environ 6 Ch.

9. Dispositif d'anse selon l'une des revendications 1 à 8, **caractérisé en ce que** l'anse (3, 4) inclut un marqueur pour rendre l'anse (3, 4) visible sous un appareil d'affichage.
